(19) Europäisches Patentamt
European Patent Office
Office européen des brevets

(11) EP 1 159 756 B1

(12) **EUROPEAN PATENT SPECIFICATION**

(45) Date of publication and mention of the grant of the patent:
**28.06.2006 Bulletin 2006/26**

(21) Application number: **99958453.5**

(22) Date of filing: **28.12.1999**

(51) Int Cl.:
***H01L 21/00*** *(2006.01)*

(86) International application number:
**PCT/IB1999/002062**

(87) International publication number:
**WO 2000/042832 (27.07.2000 Gazette 2000/30)**

# (54) COMPOUNDS FOR THE TREATMENT OF CANCER

VERBINDUNGEN FÜR KREBS-THERAPIE

COMPOSES UTILISES POUR LE TRAITEMENT DU CANCER

(84) Designated Contracting States:
**AT BE CH CY DE DK ES FI FR GB GR IE IT LI LU MC NL PT SE**

(30) Priority: **11.03.1999 SE 9900878**
**16.03.1999 SE 9900941**

(43) Date of publication of application:
**05.12.2001 Bulletin 2001/49**

(73) Proprietor: **Ardenia Investments Ltd.**
**London W1G 8DZ (GB)**

(72) Inventor: **STRECHENOK, Oleg**
**181 62 LIDINGÖ (SE)**

(74) Representative: **Holmberg, Martin Tor**
**Bergenstrahle & Lindvall AB**
**P.O. Box 17704**
**118 93 Stockholm (SE)**

(56) References cited:
**EP-A- 0 495 265**

- **E. GERMAIN ET AL.: "Enhancement of doxorubicin cytotoxicity by polyunsaturated fatty acids in the human breast tumor cell line MDA-MB-231: relationship to lipid peroxidation" INT. J. CANCER, vol. 75, no. 4, 1998, pages 578-583, XP000957908 cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 159 756 B1

Printed by Jouve, 75001 PARIS (FR)

## Description

### Field of the invention

This invention relates to new compounds, which are useful in the treatment of cancer. These compounds are used to increase the effect of conventional cytotoxic pharmaceuticals.

### Background of the invention

Cytostatic or cytotoxic compounds are widely used in the treatment of cancer. Doxorubicin is an aminoglycosidic anthracycline antibiotic and will be used as a typical representative of this group of compounds.

The cell membrane represents a physical barrier and there are some factors that determine the rate of uptake of doxorubicin. The main factors are hydrophobicity (an increase will increase the rate of uptake) and protonation degree of amino group - pKa ( a decrease will increase the rate of entry). The doxorubicin inhibits cell growth and has a marked effect on the nuclear material, which becomes non-specifically thickened, agglutinated or broken. The major binding force between doxorubicin and DNA is intercalation of the planar chromophore, stabilised by an external electrostatic binding of the positive charged amino sugar residue with negative phosphate group of DNA.

The intercalated drug molecules appear to prevent the changes in conformation of the helix, which are necessary as a preliminary to initiation of nucleic acid synthesis. The major lethal effect of doxorubicin is inhibition of nucleic acid synthesis. As consequence the drug is more active against dividing cells and the greatest effect is in the S stage of the cell cycle (Brown J.R., Adriamycin and related anthracycline antibiotics in: Progress in Medicinal Chemistry edited by G. P. Ellis and G. B. West, Elsevier/North-Holland Biomedical Press v.15, pp.125-164, 1978).

Some observations are consistent with the formation complex of electrostatic nature between the positive amino group of doxorubicin and negative phosphate group of phospholipids such as cardiolipin, phosphatidyl serine, phosphatidyl inositol and phosphatidic acid. Cardiolipin is an almost characteristic component of the inner membrane of mitochondria, which are abundant in the cardiac muscle. The pathogenesis of the mitochondrial lesions is one of the major and more specific sub-cellular changes characterizing doxorubicin cardiotoxicity. The rather selective toxicity doxorubicin for mitochondria may be due to the high concentrations of cardiolipin in the mitochondria of the cardiac muscle (Duarte-Karim M., *et al.* Biochem. Biophys. Res. Comm., v.71, N.2, pp.658-663, 1976).

The interaction between doxorubicin and lipids has been studied using large unilamellar vesicles (LUVET) composed of mixtures of anionic phospholipids and various zwitterionic phospholipids. Dilution of anionic lipids with zwitterionic lipids leads to decreased membrane association of the drug because electrostatic forces are very important in doxorubicin-membrane interaction. However, binding of doxorubicin to LUVET composed of anionic phospholipids combined with phosphatidylethanolamine (PE) is much higher than binding to LUVET made of anionic lipids plus a range of other zwitterionic lipids such as phosphatidylcholine and the N-methyethanolamine and N,N-dimethylethanolamine derivatives of PE (Speelmans G, *et al.,* Biochemistry, v.36, N.28, pp.8657-8662, 1997).

The interaction of adriamycin with human erythrocytes was investigated in order to determine the membrane binding sites and the resultant structural perturbation. Electron microscopy revealed that red blood cells incubated with the therapeutic concentration of the drug in human plasma changed their discoid shape to both stomatocytes and echinocytes. The drug was incubated with molecular models. One of them consisted of dimyristoylphosphatidylcholine and dimyristoylphosphatidylethanolamine multilayers, representatives of phospholipid classes located in the outer and inner leaflets of the erythrocyte membrane, respectively. X-ray diffraction showed that adriamycin interaction perturbed the polar head and acyl chain regions of both lipids. It is concluded that adriamycin incorporates into both erythrocyte leaflets affecting its membrane structure (Suwalsky M., Z Naturforsch [C] v.54, N3-4, pp.271-277, 1999).

The different physicochemical properties of dipalmitoylphosphatidylcholine liposomes with soybean-derived sterols have been studied. Liposomal doxorubicin increased the pharmacological effect compared with free drug, suggesting a decrease of side effect and long circulation (Maitani Y., Yakugaku Zasshi, v.116, N.12, pp.901-910, 1996).

Liposomes containing polyethylene glycol-derivatised phospholipids are able to evade the reticulo-endothelial system and thereby remain in circulation for prolonged periods. The doxorubicin encapsulated in these sterically stabilised liposomes suppresses the growth of established human lung tumour xenografts in severe combined immunodeficient mice and inhibits the spontaneous metastases of these tumours (Sakakibara T., *et al.,* Cancer Res., v.56, N:16, pp. 3743-3746, 1996).

A liposome encapsulation can protect surrounding tissue from the cytotoxic effects of the drugs after subcutaneous (s.c.) administration. Liposomes composed of "fluid-state" phospholipids only delayed the damaging effects of doxorubicin when injected s.c. Liposomes with a more rigid nature were much more effective in preventing local tissue damage over a longer period of time when administered s.c. (Oussoren C., *et al.,* Biochim. Biophys. Acta, v.1369, N.1, pp.169-172, 1998).

Exogenous polyunsaturated fatty acids modulate the cytotoxic activity of anti-cancer drugs in the human breast

cancer cell line MDA-MB-231. Among all polyunsaturated fatty acids tested, docosahexaenoic acid was the most potent in increasing doxorubicin cytotoxicity (E. Germain, *et al.,* Int. J. Cancer, v.75, pp. 578-583, 1998).

There remains a need for novel compounds and methods for the treatment of cancer. The present invention aims i.a. to increase the pharmacological activity of presently used anti-cancer drugs, such as doxorubicin, and to introduce novel approaches to the treatment of cancer.

**Short summary of the invention**

The present invention makes available new compounds according to the attached claims. Further, the present invention discloses a method of synthesis of these compounds, and a modified form of a cytostatic pharmaceutical compound; doxorubicin.

**Description of the invention**

It has been shown that the amount of lipoperoxides arise after the action of doxorubicin (DXR) in the presence of docosahexaenoic acid and oxidants, in the human breast cancer cells (line MDA-MB-231). This may endow tumour cells with metabolic characteristics that decrease their propensity to survive the effects of doxorubicin.

The present invention concerns therapeutically useful compounds and the use of the same for the treatment of cancer. The invention also relates to the synthesis of amides of all-trans-retinoic acid and 13-cis-retinoic acid with doxorubicin having the following structures:

1. N-(all-trans-retinoyl)-doxorubicin

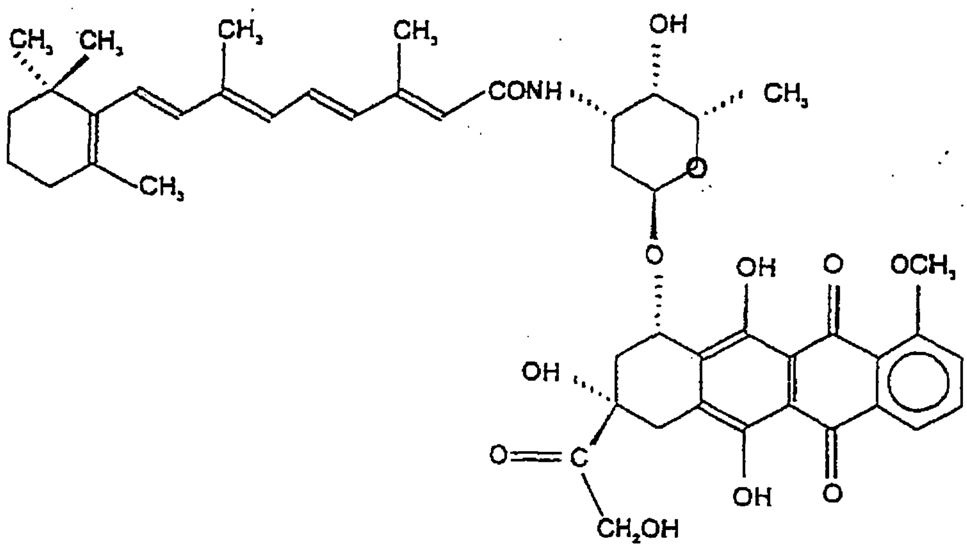

2. N-(13-cis-retinoyl)-doxorubicin

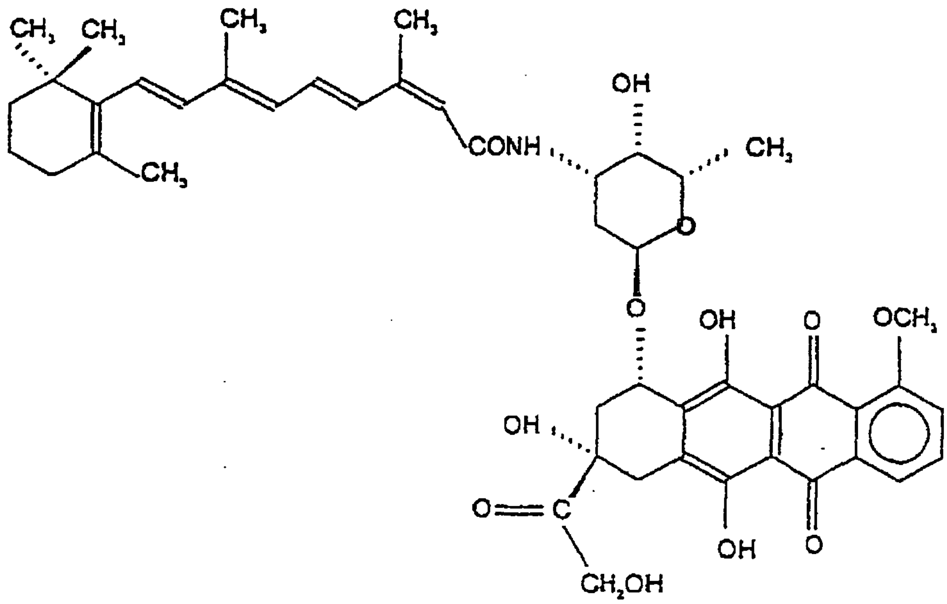

It is known that the usefulness of natural retinoids for chemoprevention is limited by their toxicity. Synthetic retinamides that possess chemopreventive activity are less toxic then the natural retinoids (Shealy Y.F., et al., J. Med. Chem. V.31., P. 190-196, 1988).
The title compounds could reveal not only higher cytotoxicity to tumor cells, but a marked diminution in the toxicity of its preparations.

Our experimental results indicate that compound 1 reveals higher antitumor activity concerning Ehrlich ascites carcinoma (EAC), passed in C3H mice. Antitumor effect of compound 1 was dose-dependent. It is evidently, that compound 1 possess higher activity then that free doxorubicin at equivalent dose. Injection of compound 1 at the dose 50 mcg/mouse (2,5 mcg/kg) inhibited tumor growth by 57% while free doxorubicin at the same dose inhibited EAC growth by 45%.
The higher cytotoxic effects the conjugates of polyunsaturated fatty acids with daunomicin to AFP- generating rat hepatoma cells could be due to high affinity of AFP to arahidonic and docosahexaenoic acids. The complex AFP with fatty acids, modified by daunomicin, was delivered to AFP-generating rat hepatoma cells, selectively.
Non AFP-producing EAC was used in our experiments. It is established that reduction of anti-tumor activity of compound 1 is caused by the presence of that protein (AFP) in the preparations, which were used for injections to mice. The result

of these experiments could be interpreted as a presence of an equilibrium reversible complex formed between AFP and retinoic acid, modified by doxorubicin. Thus higher cytotoxic activity of compound 1, in comparison with doxorubicin, could be due to its structural peculiarities. The compound 1 include two antitumor agens - amide of retinoic acids and doxorubicin.

**Example 1.**

Synthesis of N-(all-trans-retinoyl)-doxorubicin (compound 1)

all-trans-Retinoic acid (300 mg, 1 mmol) and triethylamine (104 mg, 1.02 mmol) were dissolved in 1 ml of dry tetrahydrofuran, then dry acetonitrile (4 ml) was added, and the mixture chilled to -15°C. Then 140 mg (1.02 mmol) of butyl chloroformate was added. After 30 min, the mixture free of the precipitated triethylamine hydrochloride was pipetted in a stirred suspension of Doxolem preparation containing 400 mg of doxorubicin hydrochloride and 2 g of lactose in a mixture of 1 ml of methanol and 0.1 ml triethylamine, stirring was continued for 15 min at -15°C, then the mixture obtained was allowed to warm to room temperature. After the mixture had stirred at room temperature under argon for 2 h, it was treated with 20 ml of benzene - ethanol (4:1). The suspension was centrifuged for 5 min at 3000 rpm, and the precipitate was treated with 20 ml of benzene - ethanol (4:1) and harvested by centrifugation (5 min, 3000 rpm). The two supernatants obtained were combined and evaporated to dryness in vacuo. The residue was dissolved in chloroform, and N-(all-trans-retinoyl)-doxorubicin was purified by flash chromatography on silica gel (230-400 mesh). The column was eluted successively with chloroform, acetone - chloroform (1:9, v/v), acetone - chloroform (1:4, v/v) and finally with benzene - ethanol (4:1.). The pure product was eluted with benzene - ethanol (4:1) as a deep-red band. The solvent was evaporated in vacuo to give 445 mg (78%) of N-(all-trans-retinoyl)-doxorubicin as a deep-red wax; TLC (benzene - dioxan - acetic acid 10:5:1) $R_f$ 0.5 (for doxorubicine 0.08); UV (ethanol) $A^{0.1\%}$(345 nm) = 817; $A^{0.1\%}$ (497 nm) = 196; $^1$H-NMR ($CDCl_3$, 200 MHz) δ 0.9 - 1.0 (t and s, 6H, 3 $CH_2$ ring in retinoic acid), 1.2-1.3 (d, 3H, $CH_3$-5'), 1.4 - 2.4 (m, 19H, 5$CH_3$-RA, $CH_2$-2' and $CH_2$-8), 2.9-3.3 (q, 2H, $CH_2$-10), 3.7 (s, 1H, H-4'), 3.9-4.3 (m, 6H, OH-4', $OCH_3$, H-3', H-5'), 4.6 (s, 1H, $COCH_2O$*H*), 4.8 (s, 2H, $COC$*$H_2$*OH), 5.0-5.1 (d, 1H, OH-9), 5.3 (s, 1H, H-7), 5.5 (s, 1H, H-1'), 5.6-7.0 (m, 7H, 6HC=C-RA and NH), 7.3-7.4 (d, 1H, H-3), 7.7-7.8 (t, 1H, H-2), 8.0-8.1 (d, 1H, H-1), 13.1 and 14.0 (two s, 2H, OH-6 and OH-11)

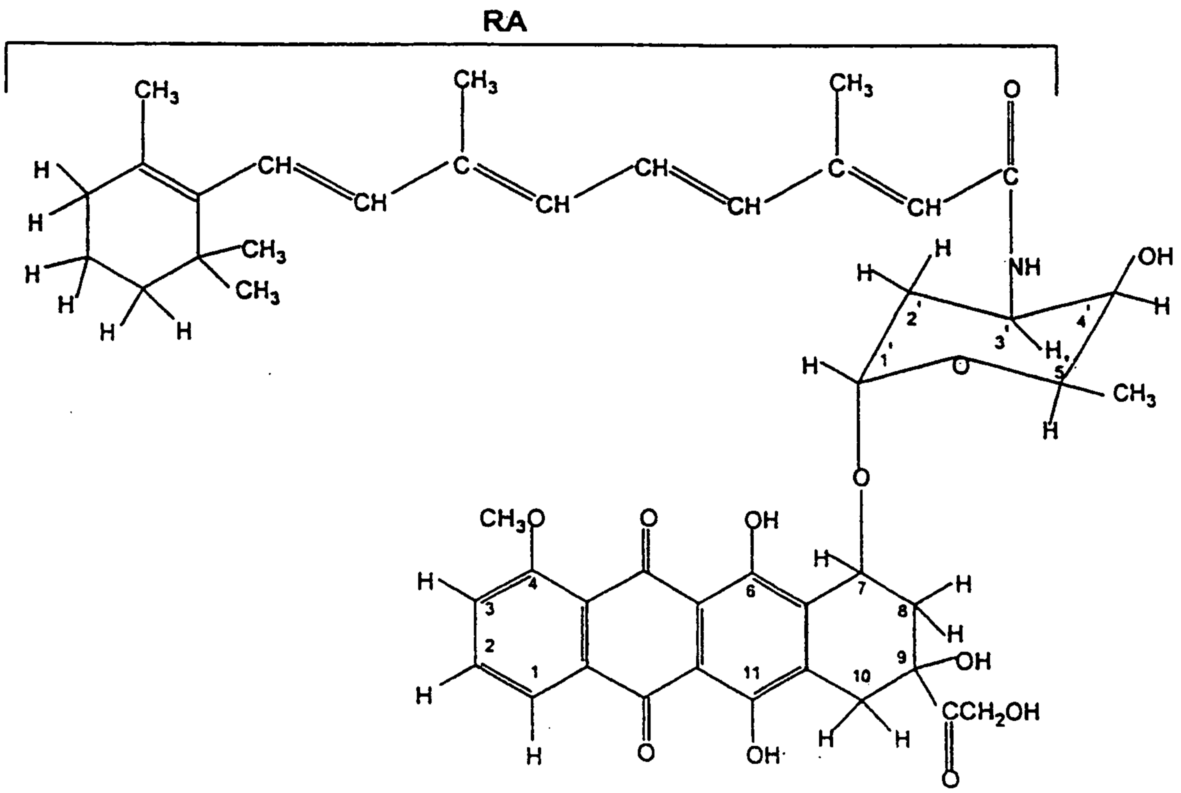

**Example 2. The antitumor effect of N-(all-trans-retinoyl)-doxorubicin (compound 1) on EAC cells**

A study of the anti-tumour effect of compound 1 as compared with doxorubicin was carried out in EAC, passed in C3H mice. 20-22 g males of C3H mice were used. Control and experimental groups included 7 animals, which were inoculated 5 x $10^6$ EAC cells i.p. in volume 0.2 ml at day O. On the second, fourth and sixth days following inoculation the doses of 20, 50 and 100 μg/mouse of compound 1, equivalent ones of doxorubicin, in 200 μl of normal mice serum (NMS) were injected i.v. Three groups of animals were administrated water solutions of doxorubicin in doses_of 1, 2.5 and 5 μg/kg of body weight (20, 50 and 100 μg/mouse by weight of 20 g accordingly). One group of mice was untreated controL Results of experiment were accounted on ninth day. Mice were killed by cervical dislocation. Ascitic fluids were removed, collected, their volumes were measured, and the abdominal cavities were washed by saline solution 6-7 times. Thus obtained fluids were pooled. The number of viable tumor cells was counted by hemocytometer, using trypan blue exclusion test. The mean and the standard mean error was calculated for each group of mice. Comparison of tumor cell number in control and experimental groups was carried out using Student t-test. Influence of tested preparation on EAC growth inhibition was evaluated by:

$$\text{Inhibition, \%} = \frac{\text{Control} - \text{Experiment}}{\text{Control}} \times 100$$

The experimental results as shown in Table 1 indicate that compound 1 displays high anti-tumor activity towards EAC. The anti-tumour effect of compound 1 is dose-dependent. Injection of compound 1 three times repeatedly on the second, fourth and sixth days following inoculation in dose of 1 mg/kg (20 μg/mouse) gives an EAC growth inhibition of 47% as compared with control untreated animals ($p < 0.01$). It is evident, that compound 1 possess a higher activity than that of free doxorubicin in an equivalent dose. In case of compound 1 injection in dose of 2.5 mg/kg (50 μg/ mouse) according to taken scheme the tumour growth inhibition reached to 56% ($p < 0.01$) while free doxorubicin at the same dose inhibited EAC growth of 45% ($p < 0.01$). The smaller effect (EAC growth inhibition of 53%) was obtained in case of injection compound 1 at dose equivalent to 5 mg/kg (100 μg/mouse) of doxorubicin. It is seen, that the cytotoxic effect of free doxorubicin at this dose is the higher than that of compound 1.

Thus, the experiment shows that compound 1 at doses, corresponding to 1 and 2.5 mg/kg of doxorubicin, exerts high anti-tumour effect towards EAC, and further, that the cytotoxicity of compound 1 is the higher than that of free doxorubicin.

**Table 1.** Antitumor effect of compound 1 and doxorubicin at three times repeated i.v. injections to mice with EAC (M ± m, n = 7)

| Group of animals | Preparation | Dose, μg/ mouse, i.v. | Ascites volume, ml | Tumor cell quantity, x $10^6$ | EAC growth inhibition, % to control | P |
|---|---|---|---|---|---|---|
| Control | Untreated | - | 5,05 ± 0,85 | 1213,6 ± 137,96 | - | - |
| Experiment 1 | Compound 1 | 20 | 3,50 ± 1,30 | 640,0 ± 78,04 | 47,3 | < 0,01 |
| Experiment 2 | Doxorubicin | 20 | 2,85 ± 1,35 | 765,8 ± 127,52 | 36,9 | < 0,05 |
| Experiment 3 | Compound 1 | 50 | 2,10 ± 1,70 | 535,0 ± 167,90 | 55,9 | < 0,01 |
| Experiment 4 | Doxorubicin | 50 | 1,85 ± 1,10 | 662,1 ± 99,17 | 45,4 | < 0,01 |
| Experiment 5 | Compound 1 | 100 | 3,30 ± 1,20 | 571,4 ± 66,96 | 52,9 | < 0,002 |
| Experiment 6 | Doxorubicin | 100 | 0,80 ± 0,60 | 350,0 ± 108,21 | 71,2 | < 0,001 |

**Example 3. Effect of N-(all-trans-retinoyl)-doxorubicin (compound 1) with AFP on growth of EAC in mice**

The influence of compound 1 with different amount of AFP on the growth of EAC was studied. The experiments were carried out on inbred C3H mice having initial weight of 20-23 g. The used tumor cell strain was supported by i.p. passages to mice of the same line. At the beginning of the research (day 0) four groups, each of which contained 7 mice, were formed. All of the animals were inoculated by tumour cells in saline solution i.p. (5 • $10^6$ cells in 0,2 ml per a mouse).

The injections of different preparations of compound 1 and doxorubicin with AFP were carried out on second,

fourth and sixth days after i.p. implantation of EAC cells to mice. The first group of animals was untreated (negative control). The mice were injected by NMS. The mice of second group were injected by compound 1 containing doxorubicin in the dose of 1 μg/kg of body weight (20 μg/mouse) i.v., NMS being used as solvent The animals of third and fourth groups were injected the same dose of compound 1, the protein being added to NMS (2,5 and 5 μg/mouse, accordingly). On the ninth day after inoculation by EAC cells the mice were killed and ascitic fluids were collected and estimated quantitatively. Tumor cells suspensions were prepared and aliquotes were mixed with equal volume of 0,1 % trypan blue solution. The quantity of tumor cells were counted with hemocytometer.

Results of the experiments were evaluated by means of variation statistics method using Student t-test. The values of inhibiting effects of the preparations were represented as percentage in comparison with control.

As seen from presented data of experiment (Table 2) compound 1 in dose of 1 mg/kg_(20 μg/mouse) inhibits the EAC growth of 45% ($p < 0{,}01$). This is in concordance with data of previous experiment In the same time the influence of compound 1 (20 μg/mouse), loaded by AFP (2,5 μg/mouse), is lowered up to 33% ($p < 0{,}01$) of tumor growth inhibition. Antitumor effect value of compound 1 with double content of AFP in injected solution (31%, $p < 0{,}02$) is not changed essentially. It is seen that effect of compound 1 with AFP (2,5 and 5,0 μg/mouse) is near to one of free doxorubicin.

**Table 2.** Effect of compound 1 in the dose of 1 mg/kg (20 μg/mouse) with AFP at three times repeated i.v. injections to mice C3H on growth of EAC (M ± m, n = 7)

| Treatment | Dose of AFP, μg/ mouse | Ascites volume, ml | Tumor cell quantity, x $10^6$ | EAC growth inhibition, % to control | p |
|---|---|---|---|---|---|
| 1. NMS | None | 3,4 ± 1,01 | 2157,8 ± 207,0 | - | - |
| 2. Compound 1 | None | 2,0 ± 0,25 | 1185,7 ± 266,2 | 45,0 | < 0,01 |
| 3. Compound 1+AFP | 2,5 | 3,0 ± 0,60 | 1442,9 ± 108,3 | 33,1 | < 0,01 |
| 4.Compound 1 +AFP | 5,0 | 2,8 ± 0,65 | 1481,4 ± 171,3 | 31,3 | < 0,02 |
| 5. Doxorubicin | None | - | - | 36,9* | < 0,01 |

* see Table 1.

**Example 4. The anti-tumour effect of N-(13-cis-retinoyl)-doxorubicin (compound 2) on growth of EAC in mice**

Mice C3H of 20-22 g were inoculated intraperitoneally with 2 x $10^6$ viable EAC cells. Starting two days later (day 2) mice were injected intravenously (in the volume of 10 ml/kg of body weight or 200 μl/mouse by weight of 20 g) once every other day, three times (day 2, 4 and 6). Mice of control group received NMS. In two groups of doxorubicin (positive controls) mice received doxorubicin alone (in NMS) in doses of 1 or 2.5 mg/kg of body weight (20 or 50 μg/ mouse by weight of 20g) accordingly. Mice of two test groups received the compound 2 in NMS, containing doxorubicin in doses of 1 or 2.5 mg/kg of body weight (20 or 50 μg/mouse by weight of 20g) accordingly. Three days later after the final treatment with test compound mice were killed by cervical dislocation on day 9. Tumour cell number was counted and the extent of inhibition of EAC growth in mice was evaluated.

Mice of control group had (975.4 ± 81.37) x $10^6$ EAC cells in abdominal cavity. In the group with doxorubicin alone in the dose of 1 mg/kg the number of tumour cells was (635.9 ± 122.78) x $10^6$ and EAC growth inhibition of 34.8 %, $p < 0.05$. In mice of test group with compound 2, containing doxorabicin in doses of 1 mg/kg, the number of tumour cells was (533.5 ± 94.23) x $10^6$ and EAC growth inhibition of 45.3 %, $p < 0.01$. In the group with doxorubicin alone in the dose of 2.5 mg/kg the number of tumour cells was (537.4 ± 92.89) x $10^6$ and EAC growth inhibition of 44.9 %, $p < 0.01$. In mice of test group with compound 2, containing doxorubicin in doses of 2.5 mg/kg, the number of tumour cells was (405.7 ± 120.62) x $10^6$ and EAC growth inhibition of 58.4 %, $p < 0.002$.

Thus, the compound 2 in doses, corresponding to 1 and 2.5 mg/kg of doxorubicin, exerts high antitumor effect in respect to EAC, exceeding the effect of doxorubicin alone.

**Example 5. Synthesis of the N-(13-cis-Retinoyl)-Doxorabicin / Compound 2/**

This compound was prepared as described above for compound 1, using 1 mmol (300mg) of 13-cis-Retinoic acid; yield 429mg (75%).

TLC (benzene -dioxan - acetic acid 10:5:1) $R_f$ 0.5 (for doxombicin 0.08);
UV (ethanol) $A^{0.1\%}$ (347 nm) = 817; $A^{0.1\%}$ (497 nm) = 196;
$^1$H-NMR ($CDCl_3$, 200 MHz) δ 0.9 - 1.0 (t and s, 6H, 3 $CH_2$ ring in retinoic acid), 1,2-2,4 (m, 22H, $CH_3$-5', 5 $CH_3$-RA, $CH_2$-

2' and $CH_2$-8), 2.9-3.3 (q, 2H, $CH_2$-10), 3.7 (s, 1H, H-4'), 3.9-4.3 (m, 6H, OH-4', $OCH_3$, H-3', H-5'), 4.6 (s, 1H, $COCH_2OH$), 4.8 (s, 2H, $COCH_3OH$), 5.0-5.1 (d, 1H, OR-9), 5.3 (s, 1H, H-7), 5.5 (s, 1H, H-1'), 5.6-7.0 (m, 7H, 6HC=C-RA and NH), 7.3-7.4 (d, 1H, H-3), 7.7-7.8 (t, 1H, H-2), 8.0-8.1 (d, 1H, H-1), 13.1 and 14.0 (two s, 2H, OH-6 and OH-11).

**Example 6. Anti-tumour effect of DXR/(C4+C4a)+C5 complexes**

(C4+C4a):C5 molar ratios were equal to 1.2:1; 1.6:1; 1.9:1 and 2.5:1. C4:C4a molar ratio was equal to 3:2. DXR - 3.5 mg/kg of body weight C5 - 6.4 mg/kg of body weight.

Mice ICR of 20-22 g were inoculated intraperitoneally with 2 x $10^6$ viable EAC cells. Starting two days later (day 2) mice were injected intravenously (in the volume of 2.5 ml/kg body weight) once every other day, three times (day 2, 4 and 6). Mice of control group received vehicle. In the group of DXR mice received DXR alone in the dose of 3.5 mg/kg. Mice of first test group received the DXR/(C4+C4a)+C5 complex. (C4+C4a):C5 molar ratio was equal to 1.2:1. Mice of second test group received the DXR/(C4+C4a)+C5 complex. (C4+C4a):C5 molar ratio was equal to 1.6:1. Mice of third test group received the DXR/(C4+C4a)+C5 complex. (C4+C4a):C5 molar ratio was equal to 1.9:1. Mice of fourth test group received the DXR/(C4+C4a)+C5 complex. (C4+C4a):C5 molar ratio was equal to 2.5:1. Two days later after the final treatment with the test complexes mice were killed by cervical dislocation on day 8. Tumour cell number was counted and the extent of inhibition of EAC growth in mice was evaluated.

Mice of control group had (768.7 $\pm$ 102.8) x $10^6$ EAC cells in abdominal cavity. In group with DXR alone the number of tumour cells was (465.1 $\pm$ 62.8) x $10^6$ and EAC growth inhibition of 39.5 %, $p < 0.05$. In mice of first test group the number of tumour cells was (283.6 $\pm$ 71.4) x $10^6$ and EAC growth inhibition of 63.1 %, $p < 0.002$. In mice of second test group the number of tumour cells was (218.3 $\pm$ 65.3) x $10^6$ and EAC growth inhibition of 71.6 %, $p < 0.001$, and with reference to DXR group (positive control) EAC growth inhibition of 53.1 %, $p < 0.02$. In mice of third test group the number of tumour cells was (299.7 $\pm$ 73.6) x $10^6$ and EAC growth inhibition of 61.0 %, $p < 0.002$. In mice of fourth test group the number of tumour cells was (405.1 $\pm$ 81.5) x $10^6$ and EAC growth inhibition of 47.3 %, $p < 0.02$.

Thus, the antitumour activity of DXR/(C4+C4a)+C5 complexes at (C4+C4a):C5 molar ratios 1.2:1; 1.6:1; 1.9: 1 and 2.5:1 exceeds the effect of DXR alone.

**Examples 7. Anti-tumour effect of DXR/(C4+C4a)+(C5+C9+C13) complexes**

(C4+C4a):(C5+C9+C13) molar ratios were equal to 1:1; 1:1.4; 1:1.8 and 1:2.3. C4:C4a molar ratio was equal to 2:3. C5:C9:C13 molar ratios were equal to 0.5:1:1. DXR - 3.5 mg/kg of body weight (C4 +C4a) - 8.15 mg/kg of body weight.

Mice ICR of 20-22 g were inoculated intraperitoneally with 2 x $10^6$ viable EAC cells. Starting two days later (day 2) mice were injected intravenously (in the volume of 2.5 ml/kg body weight) once every other day, three times (day 2, 4 and 6). Mice of control group received vehicle. In the group ofDXR mice received DXR alone in the dose of 3.5 mg/kg. Mice of first test group received the DXR/(C4+C4a)+(C5+C9+C13) complex. (C4+C4a):(C5+C9+C13) molar ratio was equal to 1:1. Mice of second test group received the DXR/(C4+C4a)+(C5+C9+C13) complex. (C4+C4a):(C5+C9+C13) molar ratio was equal to 1:1.4. Mice of third test group received the DXR/(C4+C4a)+(C5+C9+C13) complex. (C4+C4a):(C5+C9+C13) molar ratio was equal to 1:1.8. Mice of fourth test group received the DXR/(C4+C4a)+(C5+C9+C13) complex. (C4+C4a):(C5+C9+C13) molar ratio was equal to 1:2.3). Two days later after the final treatment with the test complexes mice were killed by cervical dislocation on day 8. Tumour cell number was counted and the extent of inhibition of EAC growth in mice was evaluated.

Mice of control group had (817.2 $\pm$ 99.9) x $10^6$ EAC cells in abdominal cavity. In group with DXR alone the number of tumour cells was (517.7 $\pm$ 74.9) x $10^6$ and EAC growth inhibition of 36.9 %, $p < 0.05$. In mice of first test group the number of tumour cells was (326.1 $\pm$ 92.8) x $10^6$ and EAC growth inhibition of 60.1 %, $p < 0.01$. In mice of second test group the number of tumour cells was (259.0 $\pm$ 72.6) x $10^6$ and EAC growth inhibition of 68.3 %, $p < 0.001$, and with reference to DXR group (positive control) EAC growth inhibition of 49.8 %, $p < 0.05$. In mice of third test group the number of tumour cells was (316.3 $\pm$ 81.3) x $10^6$ and EAC growth inhibition of 61.3 %, $p < 0.002$. In mice of fourth test group the number of tumour cells was (431.5 $\pm$ 74.1) x $10^6$ and EAC growth inhibition of 47.2 %, $p < 0.01$.

Thus, the anti-tumour activity of DXR/(C4+C4a)+(C5+C9+C13) complexes at (C4+C4a):(C5+C9+C13) molar ratios 1:1; 1:1.4; 1:1.8 and 1:2.3 exceeds the effect of DXR alone.

**Example 8. Antitumour effect of DXR/(C4+C4a)+(C5+C9+C13+C17) complexes**

(C4+C4a):(C5+C9+C13+C17) molar ratios were equal to 1:1.3; 1:1.6; 1:2 and 1:2.5. C4:C4a molar ratio was equal to 1:1. C5:C9:C13:C17 molar ratios were equal to 1:1:1:1. DXR - 3.5 mg/kg of body weight. (C4+C4a) - 8.15 mg/kg of body weight

Mice ICR of 20-22 g were inoculated intraperitoneally with 2 x $10^6$ viable EAC cells. Starting two days later

(day 2) mice were injected intravenously (in the volume of 2.5 ml/kg body weight) once every other day, three times (day 2, 4 and 6). Mice of control group received vehicle. In the group of DXR mice received DXR alone in the dose of 3.5 mg/kg. Mice of first test group received the DXR/(C4+C4a)+(C5+C9+C13+C17) complex (C4+C4a):(C5+C9+C13+C17) molar ratio was equal to 1:1.3. Mice of second test group received the DXR/(C4+C4a)+(C5+C9+C13+C17) complex. (C4+C4a):(C5+C9+C13+C17) molar ratio was equal to 1:1.6. Mice of third test group received the DXR/(C4+C4a)+(C5+C9+C13+C17) complex (C4+C4a):(C5+C9+C13+C17) molar ratio was equal to 1:2. Mice of fourth test group received the DXR/(C4+C4a)+(C5+C9+C13+C17) complex (C4+C4a):(C5+C9+C13+C17) molar ratio was equal to 1:2.5. Two days later after the final treatment with the test complexes mice were killed by cervical dislocation on day 8.

Tumour cell number was counted and the extent of inhibition of EAC growth in mice was evaluated.

Mice of control group had (738.1 ± 73.9) x $10^6$ EAC cells in abdominal cavity. In group with DXR alone the number of tumour cells was (431.1 ± 57.4) x $10^6$ and EAC growth inhibition of 41.6 %, $p < 0.01$. In mice of first test group the number of tumour cells was (235.9 ± 65.1) x $10^6$ and EAC growth inhibition of 68.0 %, $p < 0.001$, and with reference to DXR group (positive control) EAC growth inhibition of 45.3 %, $p < 0.05$. In mice of second test group the number of tumour cells was (223.7 ± 70.3) x $10^6$ and EAC growth inhibition of 69.7 %, $p < 0.001$ and with reference to DXR group (positive control) EAC growth inhibition of 48.1 %, $p < 0.05$. In mice of third test group the number of tumour cells was (322.6 ± 88.5) x $10^6$ and EAC growth inhibition of 56.3 %, $p < 0.01$. In mice of fourth test group the number of tumour cells was (417.2 ± 69.7) x $10^6$ and EAC growth inhibition of 43.5 %, $p < 0.01$.

Thus, the anti-tumour activity of DXR/(C4+C4a)+(C5+C9+C13+C17) complexes at (C4+C4a):(C5+C9+C13+C17) molar ratios 1:1.3; 1:1.6; 1:2 and 1:2.5 exceeds the effect of DXR alone.

## Claims

1. A compound having the following formula:

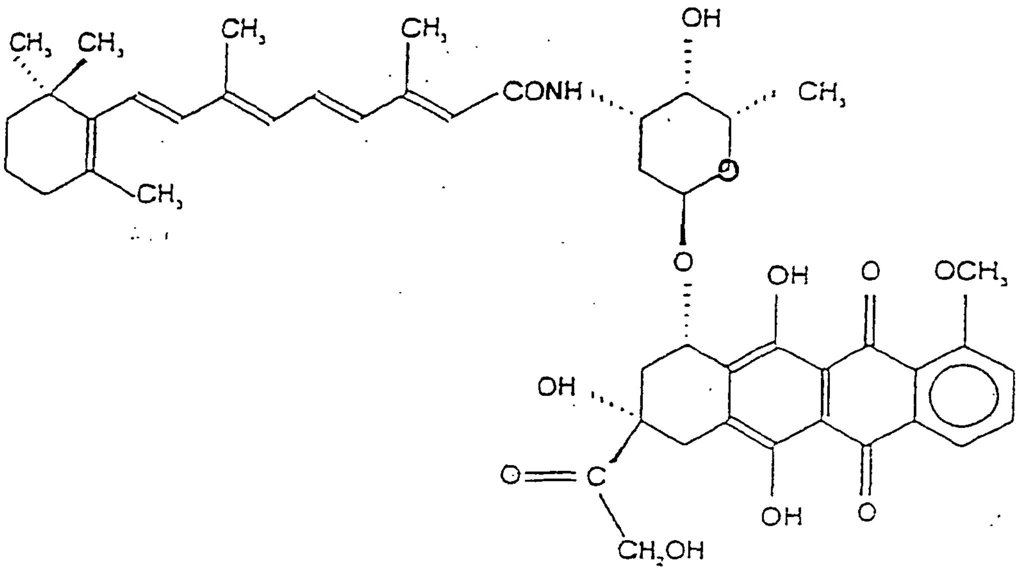

(I)

or

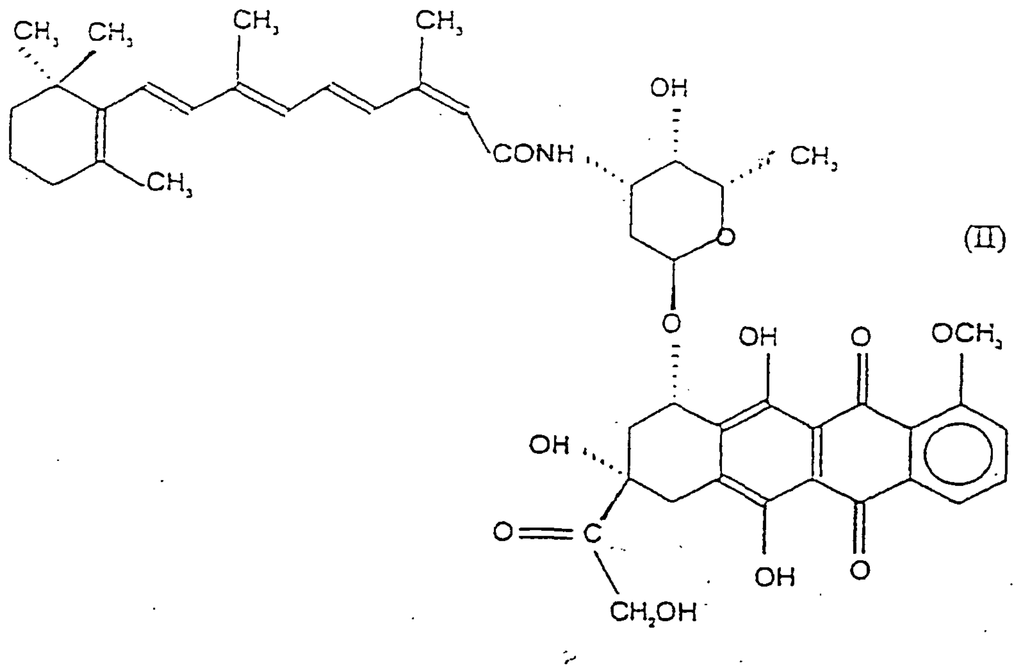

(II)

2. The use of N-(all-trans-retinoyl)-doxorubicin (I) for the manufacture of a therapeutic agent.

3. The use of N-(13-cis-retinoyl)-doxorubicin (II) for the manufacture of a therapeutic agent.

4. The use of N-(all-trans-retinoyl)-doxorubicin (I) for the manufacture of a therapeutic agent for the treatment of cancer.

5. The use of N-(13-cis-retinoyl)-doxorubicin (II) for the manufacture of a therapeutic agent for the treatment of cancer.

6. The use of N-(all-trans-retinoyl)-doxorubicin (I) for the manufacture of a therapeutic preparation to be administered intravenously for the treatment of cancer.

7. The use of N-(13-cis-retinoyl)-doxorubicin (II) for the manufacture of a therapeutic preparation to be administered intravenously for the treatment of cancer.

**Patentansprüche**

1. Eine Verbindung, die die folgende Formel besitzt:

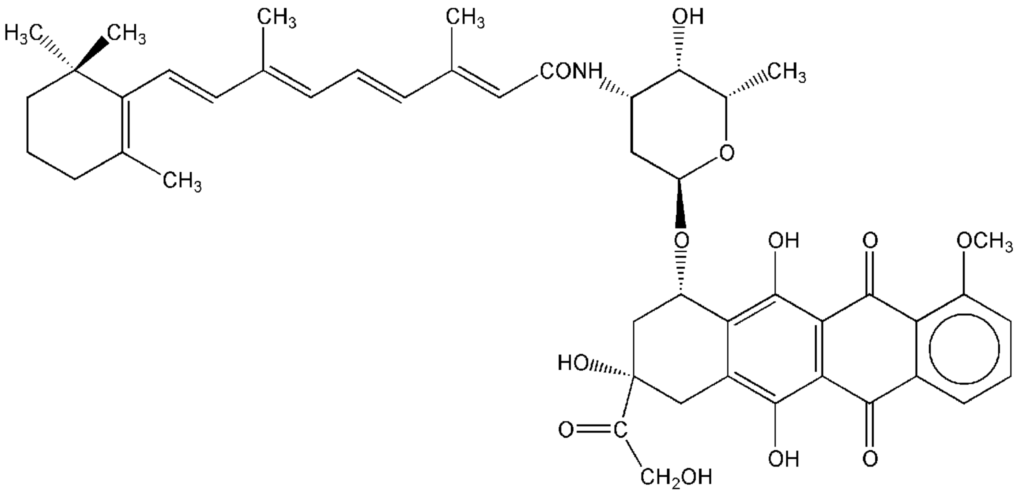

(I)

oder

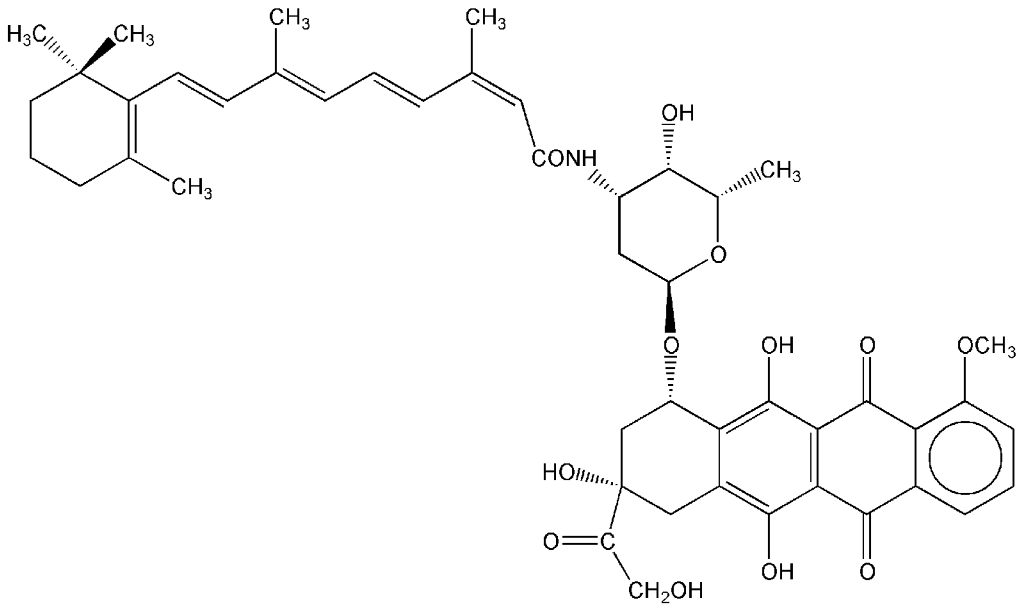

(II)

2. Die Verwendung von N-(all-trans-Retinoyl)-Doxorubicin (I) für die Herstellung eines therapeutischen Mittels.

3. Die Verwendung von N-(13-cis-Retinoyl)-Doxorubicin (II) für die Herstellung eines therapeutischen Mittels.

4. Die Verwendung von N-(all-trans-Retinoyl)-Doxorubicin (I) für die Herstellung eines therapeutischen Mittels für die Behandlung von Krebs.

5. Die Verwendung von N-(13-cis-Retinoyl)-Doxorubicin (II) für die Herstellung eines therapeutischen Mittels für die Behandlung von Krebs.

6. Die Verwendung von N-(all-trans-Retinoyl)-Doxorubicin (I) für die Herstellung eines intravenös zu verabreichenden therapeutischen Präparats für die Behandlung von Krebs.

7. Die Verwendung von N-(13-cis-Retinoyl)-Doxorubicin (II) für die Herstellung eines intravenös zu verabreichenden therapeutischen Präparats für die Behandlung von Krebs.

**Revendications**

1. Composé ayant la formule suivante :

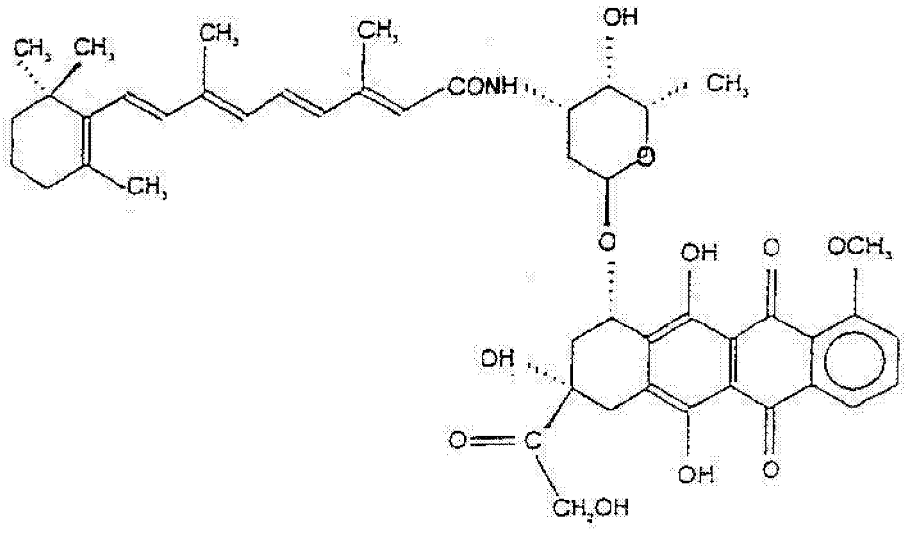

(I)

ou

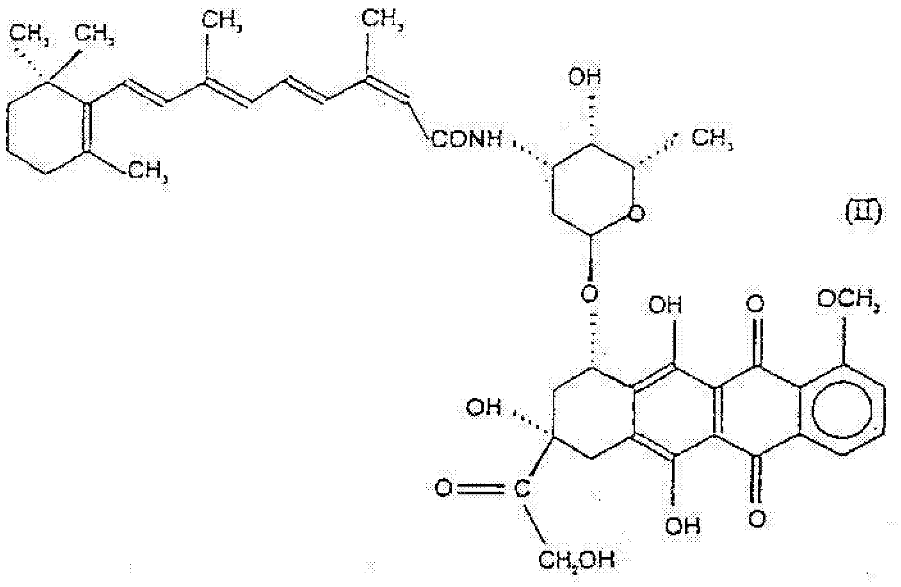

(II)

2. Utilisation de la N-(tout-trans-rétinoyl)-doxorubicine (I) pour la fabrication d'un agent thérapeutique.

3. Utilisation de la N-(13-cis-rétinoyl)-doxorubicine (II) pour la fabrication d'un agent thérapeutique.

4. Utilisation de la N-(tout-trans-rétinoyl)-doxorubicine (I) pour la fabrication d'un agent thérapeutique pour le traitement du cancer.

5. Utilisation de la N-(13-cis-rétinoyl)-doxorubicine (II) pour la fabrication d'un agent thérapeutique pour le traitement du cancer.

**6.** Utilisation de la N-(tout-trans-rétinoyl)-doxorubicine (I) pour la fabrication d'une préparation thérapeutique à administrer par voie intraveineuse pour le traitement du cancer.

**7.** Utilisation de la N-(13-cis-rétinoyl)-doxorubicine (II) pour la fabrication d'une préparation thérapeutique à administrer par voie intraveineuse pour le traitement du cancer.